(19)

Europäisches Patentamt

European Patent Office

Office européen des brevets

(11) **EP 3 020 796 A1**

(12) **EUROPÄISCHE PATENTANMELDUNG**

(43) Veröffentlichungstag:
**18.05.2016 Patentblatt 2016/20**

(51) Int Cl.:
***C12M 1/34*** *(2006.01)*

(21) Anmeldenummer: **14193507.2**

(22) Anmeldetag: **17.11.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Ionicon Analytik Gesellschaft m.b.h.**
**6020 Innsbruck (AT)**

(72) Erfinder:
 • **Gutmann, Rene**
  **6020 Innsbruck (AT)**

 • **Herbig, Jens**
  **6020 Innsbruck (AT)**
 • **Winkler, Klaus**
  **6020 Innsbruck (AT)**
 • **Luchner, Markus**
  **1170 Wien (AT)**
 • **Striedner, Gerald**
  **1220 Wien (AT)**

(74) Vertreter: **Schwarz & Partner**
**Patentanwälte**
**Wipplingerstraße 30**
**1010 Wien (AT)**

(54) **Verfahren zur Bestimmung der Aktivität von Mikroorganismen**

(57) Verfahren zur Ermittlung der Aktivität von Mikroorganismen in einer Fermentationsanlage während eines Fermentationsprozesses durch Bestimmung der Konzentration zumindest eines Metaboliten des Fermentationsprozesses, wobei der zumindest eine Metabolit ein bei Prozesstemperatur flüssiger oder fester Stoff ist, welcher in der Fermentationslösung in gelöstem Zustand einen Partialdruck im Abgas der Fermentationsanlage aufweist, wobei die Konzentration des Metaboliten während des Fermentationsprozesses im Abgas der Fermentationsanlage zu mehreren Zeitpunkten gemessen wird, wobei aus der Konzentration des Metaboliten im Abgas zum jeweiligen Zeitpunkt die Konzentration des Metaboliten in der Fermentationslösung ermittelt wird, wobei aus dem zeitlichen Verlauf der Konzentration des Metaboliten in der Fermentationslösung entweder die Produktionsrate oder die gesamt produzierte Menge eines Fermentationsproduktes ermittelt wird.

EP 3 020 796 A1

**Beschreibung**

Die vorliegende Erfindung betrifft ein Verfahren zur Ermittlung der Aktivität von Mikroorganismen in einer Fermentationsanlage während eines Fermentationsprozesses durch Bestimmung der Konzentration zumindest eines Metaboliten des Fermentationsprozesses. Ferner betrifft die Erfindung ein Verfahren zur Ermittlung der Qualität von Fermentationslösungen in einer Fermentationsanlage während eines Fermentationsprozesses durch Bestimmung der Konzentration zumindest einer flüchtigen Verbindung innerhalb des Fermentationsprozesses. Schließlich betrifft die Erfindung eine Fermentationsanlage, umfassend einen Reaktor, einen Gaszufluss und einen Abgasauslass.

Insbesondere Arzneimittel werden vermehrt durch Mikroorganismen in großen Fermentationsanlagen hergestellt. Die Kontrolle des Fermentationsvorgangs basiert weitgehend auf off-line Methoden, d.h. es wird eine Probe aus dem laufenden Prozess genommen und im Labor analysiert. Diese Vorgehensweise ist zeit- und arbeitsintensiv und hat den Nachteil, dass die Resultate meist zu spät vorliegen um noch korrigierend in den Prozess eingreifen zu können.

On-line Methoden stellen wichtige prozessrelevante Informationen zeitnah zur Verfügung, sodass eine direkte Prozessüberwachung und -kontrolle ermöglicht wird. Ein bekanntes Verfahren setzt dabei darauf, die Konzentrationen von flüchtigen Verbindungen im Gasraum der Fermentationsanlage mit hochsensitiven Messinstrumenten zu messen. Die klassische Anwendung ist die Messung von $CO_2$ und $O_2$ im Gasraum.

Eine Möglichkeit flüchtige Verbindungen im Gasraum zu messen ist mit einem Spurengasanalysator wie z.B. dem Protonen Tausch Reaktions Massenspektrometer (PTR-MS)-bio System. Ein spezieller Aufbau (-bio) ermöglicht die akkurate Messung, welcher von Luchner et al. (Luchner M., Gutmann R., Bayer K., Dunkl J., Hansel A., Herbig J., Singer W., Strobl F., Winkler K., Striedner G.: Implementation of proton transfer reaction-mass spectrometry (PTR-MS) for advanced bioprocess monitoring, Biotechnology and Bioengineering, 2012, online DOI: 10.1002/bit.24579) veröffentlicht wurde.

Der Vorteil der Gasraummessung ist, dass keine Änderungen am Prozess oder im Good Manufacturing Practise (GMP) Bereich gemacht werden müssen, denn die flüchtigen Verbindungen werden im Abluftstrom hinter der Sterilbarriere gemessen.

Die flüchtigen Verbindungen, die im Gasraum zu finden sind, sind (zum Großteil) Metaboliten, welche von den Mikroorganismen im Fermenter produziert werden. Ihre Konzentration ist daher direkt mit dem Zustand der Mikroorganismen verbunden.

Der Verlauf der gemessenen Konzentrationen kann direkt zur Überwachung der Fermentation herangezogen werden: Durch die Messung einer Vielzahl an flüchtigen Metaboliten erhält man sehr viele komplementäre Informationen über den Prozess. Gibt es während der Fermentation bei keinem der (ausgewählten) Stoffe eine Abweichung zu einer Referenzfermentation, kann man daraus schließen, dass die Fermentation gleich der Referenzfermentation, d.h. ohne Störung, verlaufen ist.

Des Weiteren lässt die Messung eine Qualitätskontrolle für flüchtige Medienbestandteile zu, wie für solche die zum Beispiel nach einer Dampfsterilisation entstehen können.

Typische Emissionsprofile können verschiedenen Mikroorganismen zugeschrieben werden und somit eine Kontamination durch Fremdorganismen frühzeitig entdeckt werden wie z.B. bei Bunge et al. (Bunge M., Araghipour N., Mikoviny T., Dunkl J., Schnitzhofer R., Hansel A., Schinner F., Wisthaler A., Margesin R., Märk T.: On-Line Monitoring of Microbial Volatile Metabolites by Proton Transfer Reaction-Mass Spectrometry, Applied and Environmental Microbiology, 2008, p. 2179-2186 Vol. 74, No. 7, doi:10.1128/AEM.02069-07).

Kurzbeschreibung der Erfindung

Bekannte Verfahren und Vorrichtungen eignen sich für die Messung von sehr flüchtigen Metaboliten, wie z.B. $CO_2$. Im Rahmen der gegenständlichen Erfindung wurde herausgefunden, dass die so gemessenen sehr flüchtigen Metaboliten eine gute in-process Kontrolle wie z.B. für bestimmte Störungen im Fermentationsprozess ermöglichen da diese direkt mit ihrer Produktionsrate (Menge produzierten Stoffes pro Zeiteinheit) korrelierten. Es wurde allerdings auch herausgefunden, dass eine Vielzahl weniger flüchtiger Metaboliten von den Mikroorganismen im Zuge einer Fermentation produziert wird, zudem zumeist in sehr geringer Konzentration. Solche weniger flüchtigen Verbindungen akkumulieren zuerst in der Fermentationslösung und treten erst dann in messbaren Konzentrationen im Gasraum auf. Der Verlauf der gemessenen Gasraumkonzentration kann daher sehr unterschiedlich im Vergleich zur Produktionsrate aussehen und die Produktion bzw. Produktionsrate eines Metaboliten daraus nicht direkt abgeleitet werden. Das ist eine mögliche Erklärung, warum bisher kaum direkte Korrelationen von solchen Verbindungen mit anderen Prozessparametern gefunden werden konnten.

Aufgabe der vorliegenden Erfindung ist es, die Produktionsrate von weniger flüchtigen Metaboliten in der Fermentationslösung zu bestimmen, um eine bessere on-line Kontrolle des Fermentationsprozesses zu ermöglichen.

Gelöst wird diese Aufgabe durch ein Verfahren zur Ermittlung der Aktivität von Mikroorganismen in einer Fermentationsanlage während eines Fermentationsprozesses durch Bestimmung der Konzentration zumindest eines Metaboliten des Fermentationsprozesses. Für zumindest einen Metaboliten, welcher bei Prozesstemperatur ein flüssiger oder fester Stoff ist und in der Fermentationslösung in gelöstem Zustand einen Partialdruck im Abgas der Fermentationsanlage aufweist, wird die Konzentration während des Fermentationsprozesses im Abgas der Fermentationsanlage zu mehreren Zeitpunkten gemessen. Aus der Konzentration des Metaboliten im Abgas wird zum jeweiligen Zeitpunkt die Konzentration des Metaboliten in der Fermentationslösung ermittelt, und in Folge aus dem zeitlichen Verlauf der Konzentration des Metaboliten die Produktionsrate eines Fermentationsproduktes bestimmt. Infolge kann bei korrelierender Produktion die Menge an produzierten nichtflüchtigen Metaboliten bestimmt werden, welcher aus demselben Stoffwechselweg stammt.

Es wird daher über die hochsensitive und hochzeitaufgelöste Messung von flüchtigen Metaboliten, welche in einem Fermentationsprozesses entstehen, eine on-line Kontrolle des Fermentationsprozesses ermöglicht, um in weiterer Folge eine Umrechnung auf die Produktionsrate von flüchtigen Metaboliten zu ermöglichen.

Die vorliegende Erfindung macht von der Erkenntnis Gebrauch, aus der Konzentration im Abgas der Fermentationsanlage die Konzentration in der Fermentationslösung zurückzurechnen und aus dem zeitlichen Verlauf auf die Produktionsrate der Mikroorganismen (Quellstärke) zu schließen. Die Erfinder haben dazu ein Modell erstellt, welches auf Henrys Gesetz aufbaut und "rückwärts" über den gemessenen Zeitverlauf die Quellstärke (Produktionsrate) bestimmt. In dieses Modell gehen die Dimensionen der Fermentationsanlage, die Parameter Belüftungsrate, Füllstand, Temperatur (alle bekannt) und eine stoffspezifische Konstante (Henry's Law Constant, HLC) ein.

Der hier verwendete Begriff flüchtige Verbindung steht für alle Stoffe mit einem ausreichenden Dampfdruck, sodass unter den gegebenen Bedingungen eine Konzentration im Gasraum entsteht, die mit einem Messgerät quantifiziert werden kann. Zum Begriff flüchtige Verbindung zählen hier flüchtige organische Verbindungen (VOCs), semiflüchtige organische Verbindungen (SVOCs) aber auch nichtorganische Verbindungen mit endlichem Dampfdruck bzw. Siedepunkt wie zum Beispiel $H_2S$. Somit sind hier alle Verbindungen gemeint die eine Verdunstungszahl bis zu circa 1200 aufweisen (http://de.wikipedia.org/wiki/Flüchtigkeit). Demzufolge ist mit dem Merkmal Partialdruck eines Stoffs im Abgas der Fermentationsanlage gemeint, dass eine messbare Menge im Abgas vorhanden ist und eine entsprechende Verdunstungszahl vorliegt.

In einem Aspekt betrifft die Erfindung eine Fermentationsanlage, die einen Reaktor, einen Gaszufluss, einen Abgasauslass, und eine Vorrichtung zur Bestimmung der Konzentration zumindest eines Stoffes im Abgas der Fermentationsanlage umfasst. Die Vorrichtung zur Bestimmung der Konzentration eines Stoffes im Abgas kann jedes Gerät sein, das sich eignet, Stoffe in Realzeit bis in geringste Konzentrationen zu messen, beispielsweise Massenspektrometer mit chemischer Ionisation wie Protonen-Transfer-Reaktions Massenspektrometer, Selective-Reagent-Ionization Massenspektrometer (SRI-MS), Selected Ion Flow Tube Massenspetrometer (SIFT-MS) aber auch Ionen-Mobilitäts Massenspektrometer (IMS). Bevorzugt kann dieses ein Protonen-Transfer-Reaktions Massenspektrometer umfassen.

| | |
|---|---|
| Fig. 1 | zeigt schematisch eine Fermentationsanlage. F ist die Begasungsrate, C_gas die Konzentration eines Metaboliten im Gas, C_hs die Konzentration dieses Metaboliten im Kopfraum, C_fl die Konzentration in der Flüssigkeit und P die Rate des abgegebenen Metaboliten der Mikroorganismen an die Lösung. |
| Fig. 2a bis 2c | zeigen die Abhängigkeit der Metabolitkonzentration in der Fermentationslösung als Funktion der Zeit von drei Metaboliten mit gleicher Produktionsrate aber unterschiedlichen Flüchtigkeiten, ausgedrückt in deren HLC. Fig. 2a zeigt einen Metaboliten mit kleiner HLC, Fig. 2b mit mittlerer HLC, Fig. 2c mit großer HLC. |
| Fig. 3a, 3b | zeigt die Konzentration eines flüchtigen Metaboliten in der Flüssigkeit (C_fl) und im Kopfraum (C_hs) für a) einen niedrigen und b) einen hohen Gasfluss. Auch bei geringem Gasfluss und daher langsamer Durchmischung des Gasraumes, sind die Konzentration in der Flüssigkeit und die Konzentration im Kopfraum nahezu identisch. Diese Tatsache wird benutzt, um die Rückrechnung zu vereinfachen. Des Weiteren sieht man die Abhängigkeit des Konzentrationsverlaufs vom Gasfluss. Bei niedrigerem Fluss stellt sich eine höhere Gleichgewichtskonzentration später ein. |
| Fig. 4 | zeigt ein Diagramm, in dem aus der Konzentration eines Metaboliten im Abgas auf die Produktionsrate (Quellstärke) rückgeschlossen wurde. Die zeitliche Verzögerung in P_Vorhersage ergibt sich aus der Vereinfachung, die in Fig. 3 beschrieben wurde. |
| Fig. 5 | zeigt ein schematisches Diagramm zur indirekten Bestimmung der Quellstärke, indem die Konzentration eines flüchtigen Metaboliten im Abgas bestimmt wird und auf die Konzentration eines nicht-flüchtigen Metaboliten in der Fermentationslösung geschlossen werden kann, wenn es bei den beiden einen metabolischen Zusammenhang gibt. |
| Fig. 6 | zeigt schematisch die indirekte Bestimmung der Quellstärke gemäß Fig. 5 anhand eines Beispiels. |
| Fig. 7 | zeigt ein Beispiel der Messung der Konzentration von Azetaldehyd im Abgas und der kumulierten (insgesamt abgegebenen Menge von Mikroorganismen) Menge von Azetaldehyd in der Lösung einer bakteriellen Fermentation (fedbatch Verfahren). |

*Methode zur Rückrechnung der Produktionsrate von flüchtigen Verbindungen von Mikroorganismen aus den Konzentrationen im Abgas der Fermentationsanlage:*

*Konzentration in der Fermentationslösung*

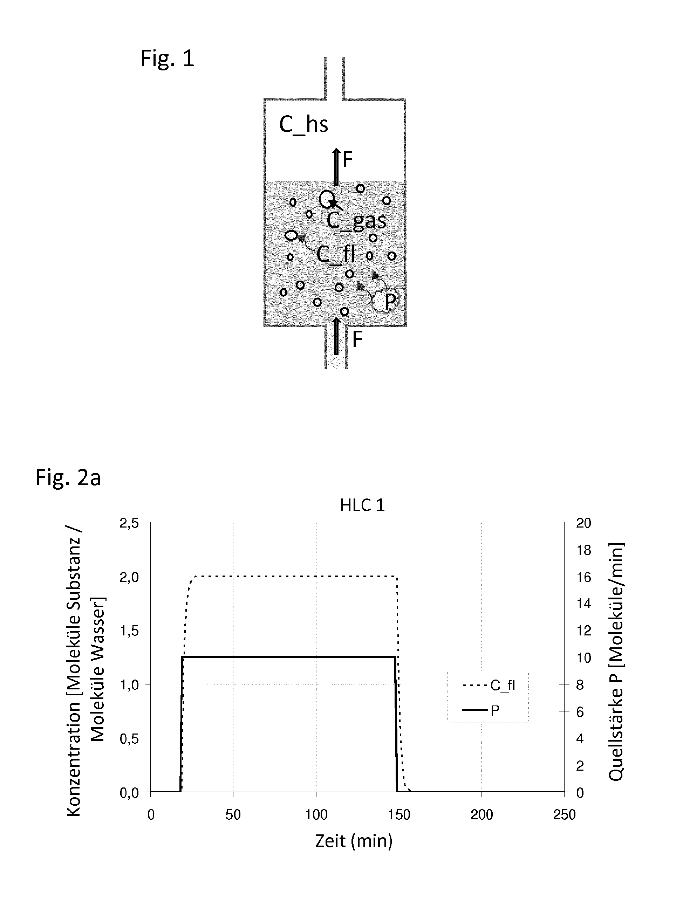

Nachfolgend wird ein Modell präsentiert, in dem die grundlegenden Zusammenhänge der Produktionsrate der Mikroorganismen für einen Metaboliten (flüchtiger Stoff; VOC) mit der messbaren Konzentration in der Abluft erläutert werden. Zur Illustration wird auf Fig. 1 hingewiesen. Die Konzentration des Metaboliten in der Fermentationslösung (C_fl) wird erhöht durch die Produktion eines Metaboliten (Produktionsrate oder Quellstärke P). Ist dieser Metabolit volatil, gast dieser aus (C_gas * F). Dies führt zu einem Verlustterm in der Fermentationslösung und einem Partialdruck im Abgas.

Das wird durch Differentialgleichung (1) beschrieben.

$$\dot{C}\_fl = \left(P - C\_gas \cdot F\right)\frac{1}{V\_fl} \qquad (1)$$

| V_fl | Volumen Wasser | in $MH_2O$ (Moleküle Wasser) |
|---|---|---|
| F | Gasfluss Luftfluss der durch den Fermenter gebubbelt wird | in Mgas/m (Moleküle Gas/ Min) |
| C_fl | Konzentration in Flüssigkeit | in $MS/MH_2O$ (Moleküle Substanz/Moleküle Wasser) |
| P | Quellstärke; Die Rate mit der die Substanz S von den Zellen in die Flüssigkeit abgegeben wird | in MS/m (Moleküle-Substanz/Min.) |
| C_gas | Konzentration in der Gasphase | in MS/Mgas |

Die Menge an Metaboliten, die ausgast, ist abhängig von der Produktionsrate und infolge von dessen Konzentration in Lösung, der HLC sowie vom Gasfluss F.

Die Rate *P* stellt sich als produzierter Metabolit pro Zeiteinheit dar und wird in den nachfolgenden Beispielen mit 10 MS (MS = Molekülen einer Substanz) pro m (m = Minute) angenommen. Diese könnte aber auch auf $\mu$g, $\mu$l, mol oder ähnliches pro Zeiteinheit umgerechnet werden.

Die Gleichgewichtskonzentration in den Gasblasen ist gegeben durch das Henry-Gesetz:

$$C\_gas = C\_fl \cdot HLC \qquad (2)$$

HLC   Henry-Konstante   in $MH_2O/Mgas$

Die HLC ist der Proportionalitätsfaktor, in den Druck und Temperatur eingehen und der angibt, wie die Gleichgewichtskonzentration eines Metaboliten im Abgas von der Konzentration in der Fermentationslösung abhängt. Die Henry-Konstante gibt es in mehreren Varianten. Eine hohe HLC bedeutet hier eine hohe Flüchtigkeit eines Metaboliten bzw. auch eine geringe Löslichkeit in der Flüssigkeit.

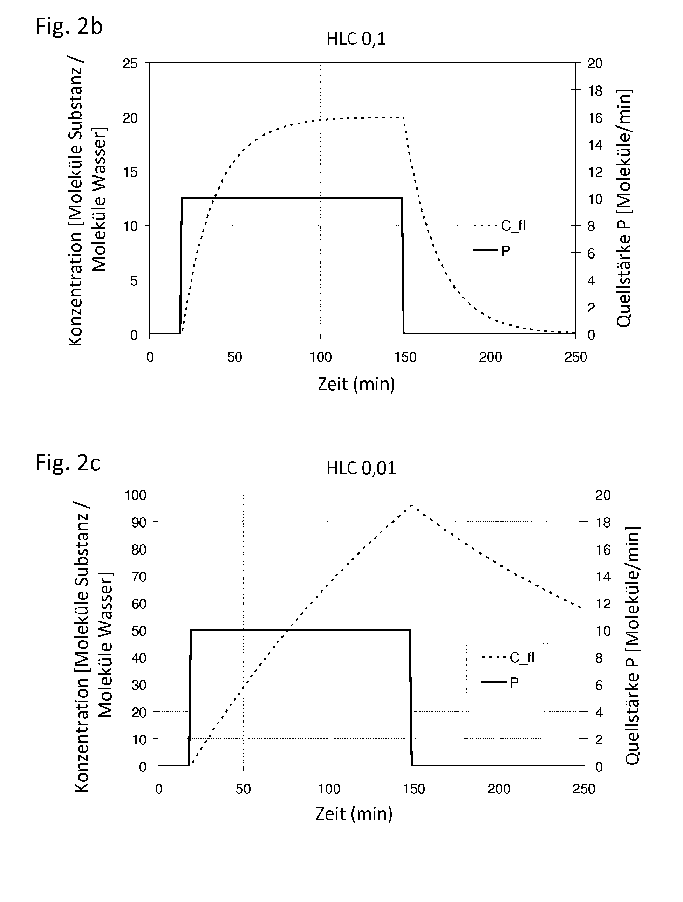

In den Beispielen der Fig. 2a, 2b und 2c erfolgt eine Gegenüberstellung von einem hypothetischen Fermentationsvorgang wobei für einen Zeitraum von Minute 20 bis Minute 150 drei Metaboliten mit verschiedenen HLCs, aber mit derselben Rate von 10 Molekülen pro Minute produziert werden. Fig. 2a zeigt den Verlauf der Konzentration des Metaboliten in Lösung mit einer großen HLC. Der Verlauf der Konzentration bildet die Produktionsrate nahezu direkt ab. Bei zunehmend geringerer HLC (Fig. 2b mittlere HLC und Fig. 2c kleine HLC) gast der Metabolit langsamer aus und akkumuliert zunehmend in der Lösung. Der Verlauf der Konzentration des Metaboliten in Lösung zeigt sich zunehmend verzerrt und bildet die Produktionsrate nicht mehr ab. Es lässt sich daher ohne Modell kein offensichtlicher Zusammenhang zwischen der Produktionsrate und der Konzentration des Metaboliten in der Lösung ableiten.

*Konzentration im Abgas:*

Die Konzentration, die aus der Flüssigkeit ausgast und die Konzentration, die sich im Abgas (Kopfraum) einstellt (C_hs) hängen folgendermaßen zusammen.

$$\dot{C}\_hs = \left(C\_gas \cdot F - C\_hs \cdot F\right)\frac{1}{V\_hs} = \left(C\_gas - C\_hs\right)\frac{F}{V\_hs} \qquad (3)$$

| | | |
|---|---|---|
| V_hs | Volumen Kopfraum | in Mgas (Moleküle Gas) |
| C_hs | Konzentration Kopfraum | in MS/Mgas (Moleküle Substanz/Moleküle Gas) |

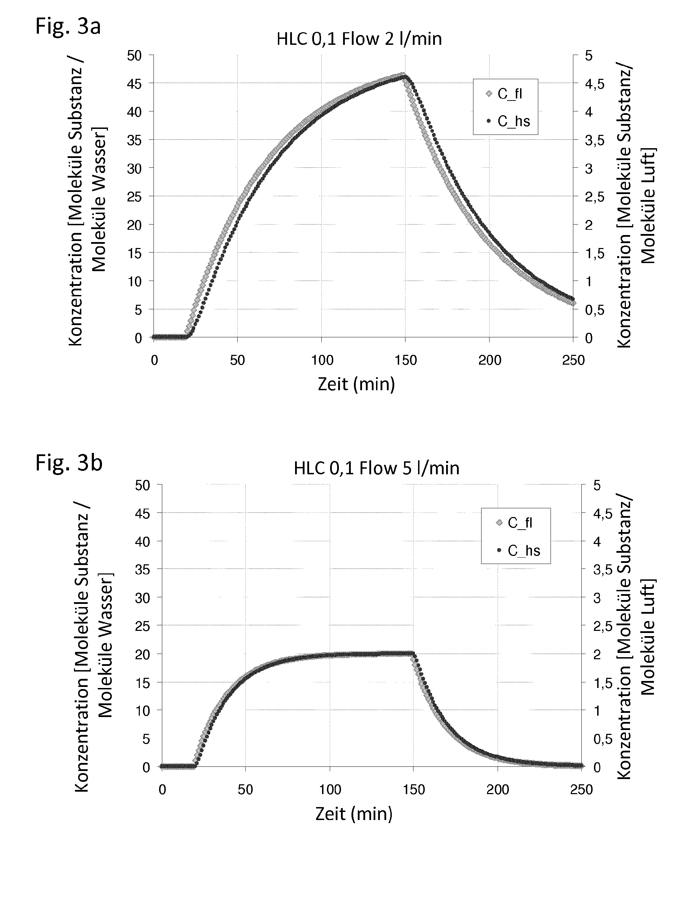

In V_hs geht der Druck ein (ideales Gasgesetz). Wie aus den Fig. 3a und 3b ersichtlich ist, führt eine niedrigere Flussgeschwindigkeit zu einer Verzögerung, also bis die Konzentration im Abgas auch im Analysengerät ankommt. Fig. 3a zeigt ein Beispiel mit Flussgeschwindigkeit von 2 l/min (Füllstand 10 l, Kopfraum 10 l), was zu einer Verzögerung von 10 Minuten führt. In Fig. 3b beträgt die Flussgeschwindigkeit 5 l / min (Füllstand 10 l, Kopfraum 10 l), was zu einer Verzögerung von 2 Minuten führt. Bei hohem Fluss und/oder kleinem Kopfraum sind die Konzentrationen nahezu identisch. Wenn die Konzentration C_gas gleich der Konzentration im Kopfraum ist, was sich aus Gleichung (3) ergibt, wenn V_hs klein und/oder F groß ist, dann ist die Verzögerung durch den Kopfraum vernachlässigbar. Zur Vereinfachung der Rückrechnung kann diese Näherung ausgenutzt werden.

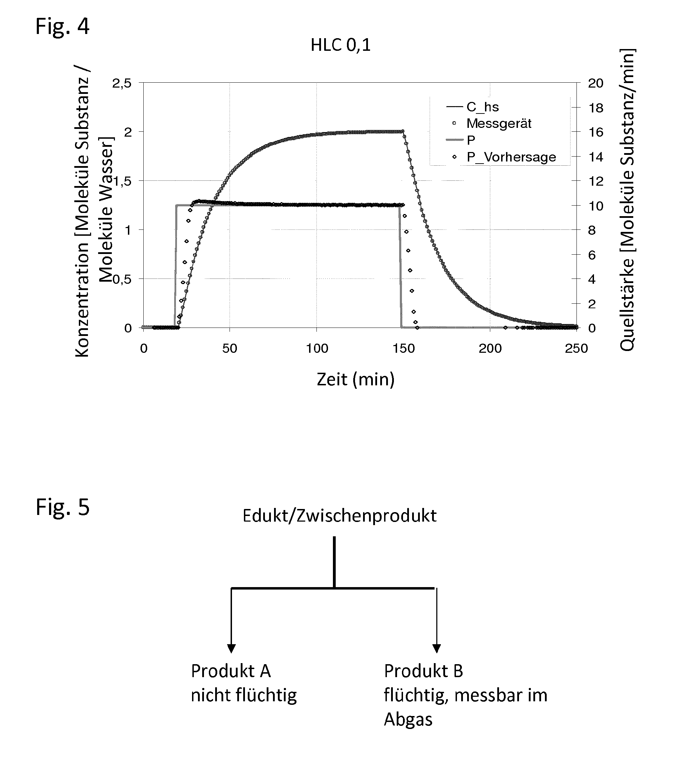

Bei sehr flüchtigen Substanzen wie $CO_2$ (oder $O_2$; Abbildung 2a) ist der Verlauf der Abgaskonzentration direkt mit der Produktionsrate korreliert. Hingegen hätte ein gut löslicher Stoff, z.B. mit der HLC von Aceton, einen sehr unterschiedlichen Verlauf von Produktionsrate und der gemessenen Konzentration (ähnlich Fig. 2 b) und es wäre keine direkte Korrelation messbar. Eine Rückrechnung ist aber über das Modell möglich. Simulation und Experiment passen gut überein, wie in Fig. 4 dargestellt.

*Rückrechnung auf die Qarellstärke:*

Die Quellstärke kann daher rückgerechnet werden. Zunächst löst man Gleichung (1) nach P auf und drückt dann mit Gleichung (2) alles als Gaskonzentration C_gas aus.

$$P = \frac{V\_fl}{H} \cdot \dot{C}\_gas + F \cdot C\_gas \qquad (4)$$

*Methode zur indirekten Bestimmung nicht-flüchtiger Metaboliten*

Im Folgenden beschreiben wir eine erweiterte Anwendung des oben beschriebenen Modells. Wenn zwei Stoffe metabolisch verbunden sind, z.B. beide im gleichen Metabolismus produziert werden, sind auch ihre Quellstärken verbunden. Wie oben beschrieben, kann durch die Messung eines flüchtigen Stoffes dessen Quellstärke berechnet werden. Dadurch kann die Quellstärke des verbundenen nicht-flüchtigen Stoffs aus dem gleichen Metabolismus berechnet werden (siehe Fig. 5).

Aus einem Edukt entsteht infolge einer metabolischen Reaktion ein Produkt A welches nicht flüchtig ist (aber wichtige Information birgt). Im Zuge der Reaktion entsteht des Weiteren ein Produkt B welches flüchtig ist, dessen Quellstärke über die Abgasmessung quantifiziert werden kann. Wenn das Verhältnis der Quellstärken konstant ist, wird eine indirekte Bestimmung des nicht flüchtigen Produktes möglich.

*Beispiel: Azetaldehyd und Azetat bei E. coli*

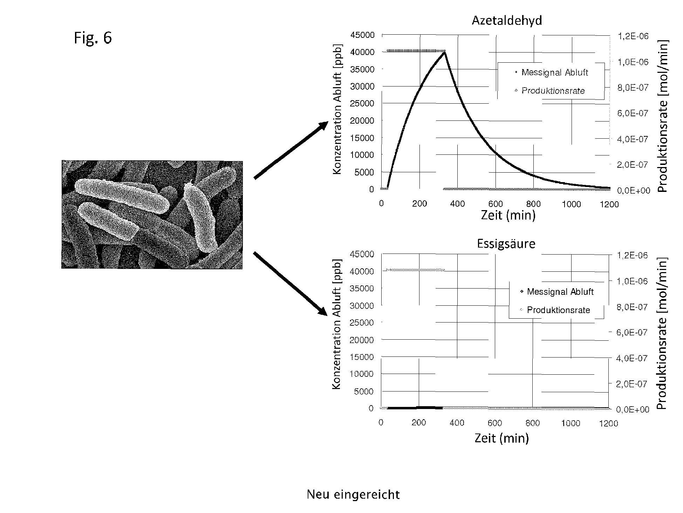

Im Zuge des Fermentationsprozesses vom E.Coli Stamm HMS174(DE3) mit dem Produkt SOD (Superoxiddismutase, ein Enzym) werden verschiedenste Stoffwechselprodukte gebildet. Laut Metabolismus (bekannt und erforscht, siehe z.B. ECOCYC, eine Datenbank für E.Coli K12; www. http://ecocyc.org/) wird unter anderem überschüssige Glucose in Azetat umgewandelt. Hohe Azetatmengen stören wiederum die Fermentation, da es ist für Bakterien toxisch ist. Eine direkte Azetatmessung über die Gasphase ist nicht möglich. Azetat hat keinen Dampfdruck, und die korrespondierende Säure, Essigsäure, nur einen sehr geringen. Da üblicherweise bei neutralen Bedingungen (pH Wert = 7) fermentiert wird liegt somit der Hauptteil der Verbindung als nicht flüchtiges Azetat vor (circa 99 %). Es ist prinzipiell möglich Essigsäure über die Gasphase zu messen aber starke Wechselwirkung mit den in Kontakt tretenden Oberflächen (Fermenter, Probengasleitung) führen zu einer Verfälschung des Messergebnisses. Laut Metabolismus (ECOCYC) wird aus Glucose über Acetyl-CoA, neben Azetat auch Azetaldehyd produziert (siehe dazu Fig. 6; Die Zellen produzieren Azetat und Azetaldehyd im gleichen Verhältnis, allerdings sehen die im Kopfraum gemessenen Signale unterschiedlich aus, da Azetat praktisch nicht flüchtig ist). Die Menge an Azetaldehyd kann über die Fermenterabluft gemessen werden. Aus der Konzentration von Azetaldehyd kann auf die Produktionsrate für Essigsäure geschlossen werden.

*Beispiel: Bestimmung von Azetat in der Fermentationsbrühe aus cler Abluft: Methode zur Berechnung der gesamt produzierten Menge (Integral) eines flüchtigen Metaboliten von Mikroorganismen aus dem Signal in der Fermenterabluft-Konzentration (gemessen mit PTR-MS)*

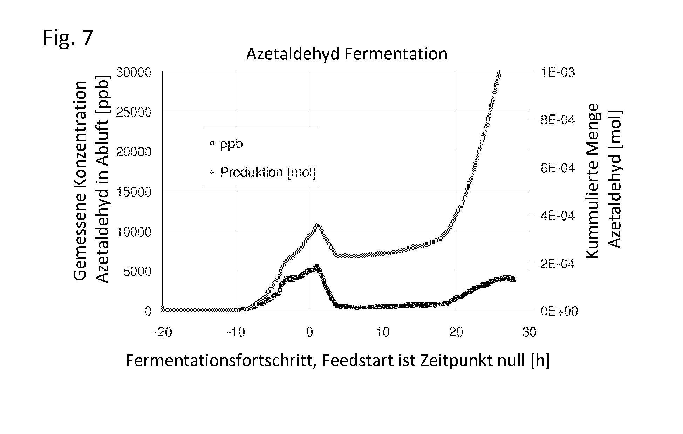

Für den nichtflüchtigen Stoff Azetat liegen reale offline gemessene Konzentrationswerte vor. Mit dem oben beschriebenen Zusammenhang kann die akkumulierte Azetatmenge in der Fermentationslösung aus der Quellstärke von Azetaldehyd berechnet werden (und umgekehrt), die in der Abluft gemessen wurde. Das Integral über die Produktionsrate ergibt die gesamte produzierte Menge des nicht flüchtigen Stoffs, der in der Flüssigkeit akkumuliert. Fig. 7: Über die Modellrechnung kann aus der momentan real gemessenen Azetaldehydkonzentration (dunkel) die Gesamtmenge an produzierten Azetaldehyd bestimmt werden (hell).

| Fermentation | Korrelation der off-line bestimmten Acetatkonzentration mit | |
| --- | --- | --- |
| | Azetaldehyd Abluft | Azetaldehyd Gesamtmenge |
| 1 | 0.91 | 0.97 |
| 2 | 0.91 | 0.96 |
| 3 | 0.66 | 0.90 |
| 4 | 0.85 | 0.91 |

Tabelle 1: Korrelationskoeffizienten von Azetaldehyd aus der Abluftmessung bzw. der Azetaldehyd-Gesamtmenge mit der Azetatmenge (offline mit anderer Methode gemessen) in der Fermentationsanlage. Die Azetaldehyd Gesamtmenge korreliert wesentlich besser als die mit direkt in der Abluft gemessenen Azetaldehyd Konzentration (Ergebnis aus 4 E-Coli Fermentationen).

Azetat wird infolge Glucoseüberschuss gebildet (mixed acid fermentation). Azetat, in größeren Konzentrationen, wirkt toxisch auf die Bakterien, sie sterben ab. In der Praxis wird Azetat offline gemessen um dann bei einer weiteren, identen Fermentation die Glucosezufuhr sowie die Induktorstärke (führt zur Produktion des gewünschten Produktes) angepasst. Die Produktion von Azetat wird dadurch minimiert. Durch den beschriebenen Zusammenhang ist es mit dieser Methode nun möglich Azetat Konzentrationen in Realzeit zu erhalten, um dann mit dieser Information mittels Änderung von Prozessparametern wie z.B. Glucosezufuhr oder Induktormenge direkt den Prozess zu optimierten.

Durch die gleichzeitige Messung mit alternativen analytischen Methoden werden Korrelationen gefunden. Zum Beispiel kann durch Korrelation mit $\mu$-array Daten (aufwendige Analytik, offline und langwierig) festgestellt werden wann Stoffwechselwege bzw. Vorgänge anspringen. Durch die Messung des VOCs in Echtzeit wird diese indirekte Information online zugänglich.

## Patentansprüche

1. Verfahren zur Ermittlung der Aktivität von Mikroorganismen in einer Fermentationsanlage während eines Fermentationsprozesses durch Bestimmung der Konzentration zumindest eines Metaboliten des Fermentationsprozesses, **dadurch gekennzeichnet, dass** der zumindest eine Metabolit ein bei Prozesstemperatur flüssiger oder fester Stoff ist, welcher in der Fermentationslösung in gelöstem Zustand einen Partialdruck im Abgas der Fermentationsanlage aufweist, wobei die Konzentration des Metaboliten während des Fermentationsprozesses im Abgas der Fermentationsanlage zu mehreren Zeitpunkten gemessen wird, wobei aus der Konzentration des Metaboliten im Abgas zum jeweiligen Zeitpunkt die Konzentration des Metaboliten in der Fermentationslösung ermittelt wird, wobei aus dem zeitlichen Verlauf der Konzentration des Metaboliten in der Fermentationslösung bzw. aus dem zeitlichen Verlauf der Konzentration des Metaboliten im Abgas entweder die Produktionsrate oder die gesamt produzierte Menge eines Fermentationsproduktes ermittelt wird.

2. Verfahren zur Ermittlung der Qualität von Fermentationslösungen in einer Fermentationsanlage während eines Fermentationsprozesses durch Bestimmung der Konzentration zumindest einer Verbindung innerhalb des Fermentationsprozesses, **dadurch gekennzeichnet, dass** diese zumindest eine Verbindung ein bei Prozesstemperatur flüssiger oder fester Stoff ist, welcher in der Fermentationslösung in gelöstem Zustand einen Partialdruck im Abgas

EP 3 020 796 A1

der Fermentationsanlage aufweist, wobei die Konzentration während des Fermentationsprozesses im Abgas der Fermentationsanlage zu mehreren Zeitpunkten gemessen wird, wobei aus der Konzentration der Verbindung im Abgas zum jeweiligen Zeitpunkt die Konzentration der Verbindung in der Fermentationslösung ermittelt wird, wobei aus dem zeitlichen Verlauf der Konzentration des Metaboliten in der Fermentationslösung die Produktionsrate oder gesamt produzierte Menge einer Verbindung ermittelt wird.

3. Verfahren nach Anspruch 1, **dadurch gekennzeichnet, dass** aus der Produktionsrate eines flüchtigen Fermentationsproduktes auf die Produktionsrate eines nicht flüchtigen Fermentationsproduktes geschlossen wird.

4. Verfahren nach Anspruch 1, **dadurch gekennzeichnet, dass** aus der gesamt produzierten Menge eines flüchtigen Fermentationsproduktes auf die gesamt produzierte Menge eines nicht flüchtigen Fermentationsproduktes geschlossen wird.

5. Verfahren nach Anspruch 4, **dadurch gekennzeichnet, dass** der im Abgas gemessene Metabolit Azetaldehyd und das Fermentationsprodukt Azetat ist.

6. Verfahren nach einem der Ansprüche 1 bis 4 **dadurch gekennzeichnet, dass** der im Abgas gemessene Metabolit Methylthiol und ein Fermentationsprodukt eine schwefelhaltige Aminosäure, schwefelhaltiges Peptid oder schwefelhaltiges Protein ist.

7. Verfahren nach einem der Ansprüche 1 bis 4, **dadurch gekennzeichnet, dass** durch die Änderung der Produktionsrate oder gesamt produzierten Menge eines flüchtigen Stoffes auf Änderungen im Stoffwechsel geschlossen wird.

8. Verfahren nach einem der Ansprüche 1 bis 7, **dadurch gekennzeichnet, dass** die Produktionsrate bzw. gesamt produzierte Menge rechnerisch bestimmt sowie der Konzentrationsverlauf eines flüchtigen Stoffes zur Regulierung eines Fermentationsprozesses mit Mikroorganismen verwendet wird, wobei in Abhängigkeit von der Rate bzw. Menge eines flüchtigen Stoffes ein die Fermentation beeinflussender Parameter verändert wird.

9. Verfahren nach Anspruch 8, **dadurch gekennzeichnet, dass** der die Fermentation beeinflussende Parameter ausgewählt ist aus der Gruppe pH-Wert, Temperatur, Sauerstoffzufuhr, Stickstoffzufuhr, Glukosegehalt, Nährstoffzufuhr, Induktorzufuhr oder Rührereinstellungen in der Fermentationsanlage.

10. Fermentationsanlage, die einen Reaktor, einen Gaszufluss, einen Abgasauslass, und eine Vorrichtung zur Bestimmung der Konzentration zumindest eines Stoffes im Abgas der Fermentationsanlage umfasst.

7

## Fig. 1

## Fig. 2a

Fig. 2b

Fig. 2c

Fig. 3a

HLC 0,1 Flow 2 l/min

Fig. 3b

HLC 0,1 Flow 5 l/min

Fig. 4

HLC 0,1

Fig. 5

Edukt/Zwischenprodukt

Produkt A
nicht flüchtig

Produkt B
flüchtig, messbar im
Abgas

Fig. 6

Azetaldehyd

- Messignal Abluft
- Produktionsrate

Zeit (min)

Essigsäure

- Messignal Abluft
- Produktionsrate

Zeit (min)

Neu eingereicht

Fig. 7

# EP 3 020 796 A1

**Europäisches Patentamt**
**European Patent Office**
**Office européen des brevets**

## EUROPÄISCHER RECHERCHENBERICHT

Nummer der Anmeldung

EP 14 19 3507

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | MARKUS LUCHNER ET AL: "Implementation of proton transfer reaction-mass spectrometry (PTR-MS) for advanced bioprocess monitoring", BIOTECHNOLOGY AND BIOENGINEERING, Bd. 109, Nr. 12, 3. Dezember 2012 (2012-12-03), Seiten 3059-3069, XP055177268, ISSN: 0006-3592, DOI: 10.1002/bit.24579 * Abbildung 5b; Tabelle 2 * * Seite 3067, linke Spalte, Zeile 10 - rechte Spalte, Zeile 10 * ----- | 1-10 | INV. C12M1/34 |
| X | LUCHNER M ET AL: "Real-time approach", EBR - EUROPEAN BIOPHARMACEUTICAL REVIEW WINTER 201 SAMEDAN LTD GBR, Nr. WINTER, Januar 2014 (2014-01), Seiten 52-55, XP009183303, ISSN: 1369-0663 * Abbildung 1 * * Seite 53, rechte Spalte, letzter Absatz * ----- | 1-5,7-10 | |
| X | US 2011/059476 A1 (SHIN HYE-WON [US] ET AL) 10. März 2011 (2011-03-10) * Absatz [0038]; Abbildungen 1A, 1B * * Absatz [0049]; Abbildung 2A * ----- | 1,2,10 | RECHERCHIERTE SACHGEBIETE (IPC)<br><br>C12M |

Der vorliegende Recherchenbericht wurde für alle Patentansprüche erstellt

| Recherchenort | Abschlußdatum der Recherche | Prüfer |
|---|---|---|
| München | 20. März 2015 | Lanzrein, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

14

## ANHANG ZUM EUROPÄISCHEN RECHERCHENBERICHT
## ÜBER DIE EUROPÄISCHE PATENTANMELDUNG NR.

EP 14 19 3507

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-03-2015

| Im Recherchenbericht angeführtes Patentdokument | Datum der Veröffentlichung | Mitglied(er) der Patentfamilie | Datum der Veröffentlichung |
|---|---|---|---|
| US 2011059476 A1 | 10-03-2011 | KEINE | |

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LUCHNER M. ; GUTMANN R. ; BAYER K. ; DUNKL J. ; HANSEL A. ; HERBIG J. ; SINGER W. ; STROBL F. ; WINKLER K. ; STRIEDNER G.** Implementation of proton transfer reaction-mass spectrometry (PTR-MS) for advanced bioprocess monitoring. *Biotechnology and Bioengineering,* 2012

- **BUNGE M. ; ARAGHIPOUR N. ; MIKOVINY T. ; DUNKL J. ; SCHNITZHOFER R. ; HANSEL A. ; SCHINNER F. ; WISTHALER A. ; MARGESIN R. ; MÄRK T.** On-Line Monitoring of Microbial Volatile Metabolites by Proton Transfer Reaction-Mass Spectrometry. *Applied and Environmental Microbiology,* 2008, vol. 74 (7), 2179-2186